# EUROPEAN PATENT SPECIFICATION

(11) **EP 2 094 350 B1**
(45) Date of publication and mention of the grant of the patent: **13.06.2018**
(21) Application number: 07865338.3
(22) Date of filing: 06.12.2007
(51) Int. Cl.: A61N 1/05

(54) **GROUPED LEADS FOR SPINAL STIMULATION**
GRUPPIERTE ELEKTRODEN FÜR RÜCKENMARKSSTIMULATION
CONDUCTEURS GROUPÉS POUR STIMULATION SPINALE

(30) Priority: 06.12.2006 US 873464 P
(43) Date of publication of application: 02.09.2009
(73) Proprietor: Spinal Modulation, Inc., Menlo Park, CA 94025 (US)
(72) Inventor: IMRAN, Mir A., Los Altos, CA 94022 (US)
(74) Representative: Booth, Catherine Louise
(86) International application number: PCT/US2007/086693
(87) International publication number: WO 2008/070804

(56) References cited:
- WO-A2-2006/084635
- US-A1- 2003 195 602
- US-A1- 2005 090 885
- US-A1- 2005 222 647
- US-A1- 2006 052 839
- US-A1- 2006 206 118
- US-B2- 6 902 547

## Description

### BACKGROUND OF THE INVENTION

The application of specific electrical energy to the spinal cord for the purpose of managing pain has been actively practiced since the 1960s. It is known that application of an electrical field to spinal nervous tissue can effectively mask certain types of pain transmitted from regions of the body associated with the stimulated nervous tissue. Such masking is known as paresthesia, a subjective sensation of numbness or tingling in the afflicted bodily regions. Application of electrical energy has been based on the gate control theory of pain. Published in 1965 by Melzack and Wall, this theory states that reception of large nerve fiber information, such as touch, sense of cold, or vibration, would turn off or close the gate to reception of painful small nerve fiber information. The expected end result would, therefore, be pain relief. Based on the gate control theory, electrical stimulation of large fibers of the spinal cord cause small fiber information to be reduced or eliminated at that spinal segment and all other information downstream from that segment would be reduced or eliminated as well. Such electrical stimulation of the spinal cord, once known as dorsal column stimulation, is now referred to as spinal cord stimulation or SCS.

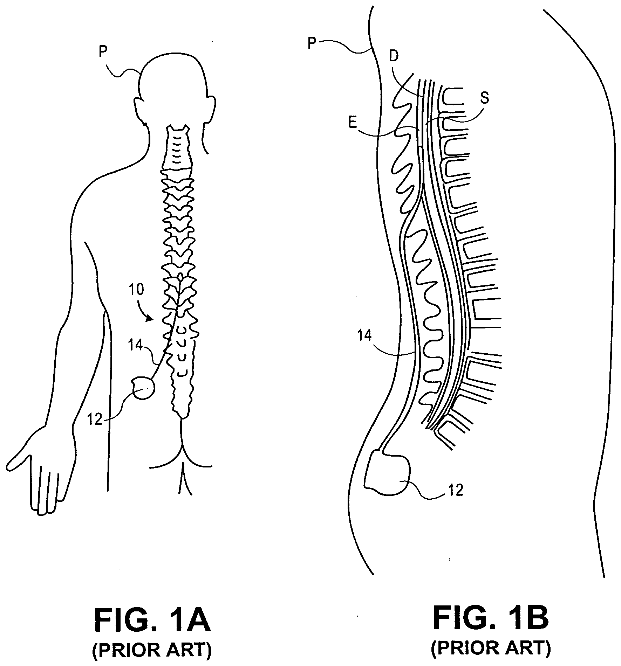

Figs. 1A-1B illustrate conventional placement of an SCS system 10. Conventional SCS systems include an implantable power source or implantable pulse generator (IPG) 12 and an implantable lead 14. Such IPGs 12 are similar in size and weight to pacemakers and are typically implanted in the buttocks of a patient P. Using fluoroscopy, the lead 14 is implanted into the epidural space E of the spinal column and positioned against the dura layer D of the spinal cord S, as illustrated in Fig. 1B. The lead 14 is implanted either through the skin via an epidural needle (for percutaneous leads) or directly and surgically through a mini laminotomy operation (for paddle leads).

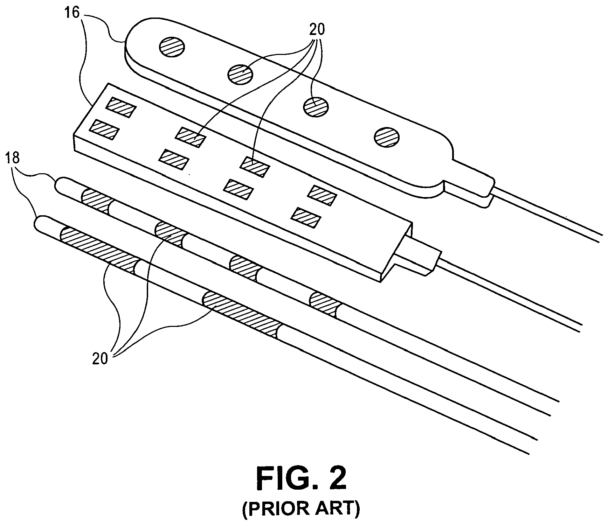

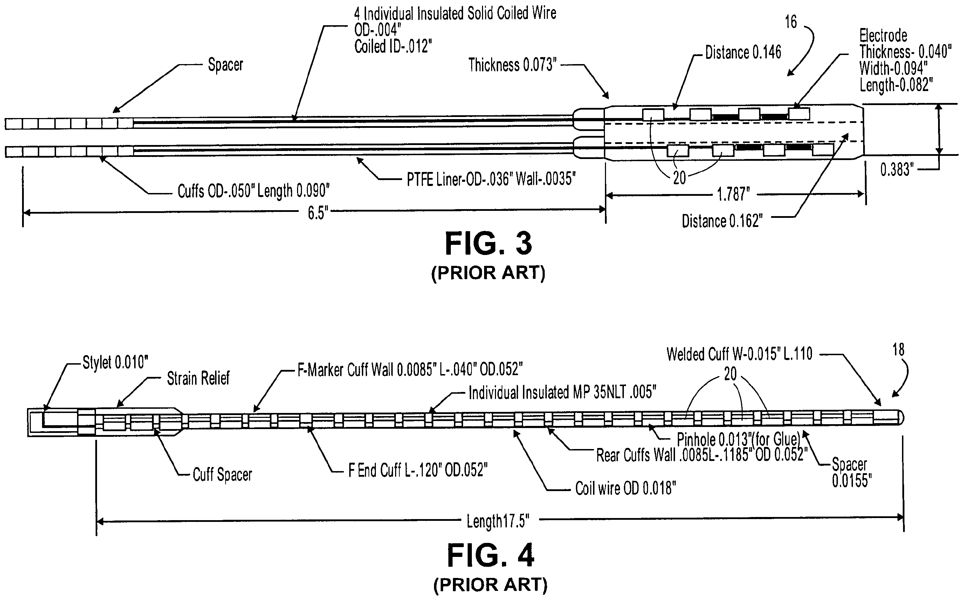

Fig. 2 illustrates example conventional paddle leads 16 and percutaneous leads 18. Paddle leads 16 typically have the form of a slab of silicon rubber having one or more electrodes 20 on its surface. Example dimensions of a paddle lead 16 is illustrated in Fig. 3. Percutaneous leads 18 typically have the form of a tube or rod having one or more electrodes 20 extending therearound. Example dimensions of a percutaneous lead 18 is illustrated in Fig. 4.

Implantation of a percutaneous lead 18 typically involves an incision over the low back area (for control of back and leg pain) or over the upper back and neck area (for pain in the arms). An epidural needle is placed through the incision into the epidural space and the lead is advanced and steered over the spinal cord until it reaches the area of the spinal cord that, when electrically stimulated, produces a comfortable tingling sensation (paresthesia) that covers the patient's painful area. To locate this area, the lead is moved and turned on and off while the patient provides feedback about stimulation coverage. Because the patient participates in this operation and directs the operator to the correct area of the spinal cord, the procedure is performed with local anesthesia.

Implantation of paddle leads 16 typically involves performing a mini laminotomy to implant the lead. An incision is made either slightly below or above the spinal cord segment to be stimulated. The epidural space is entered directly through the hole in the bone and a paddle lead 16 is placed over the area to stimulate the spinal cord. The target area for stimulation usually has been located before this procedure during a spinal cord stimulation trial with percutaneous leads 18.

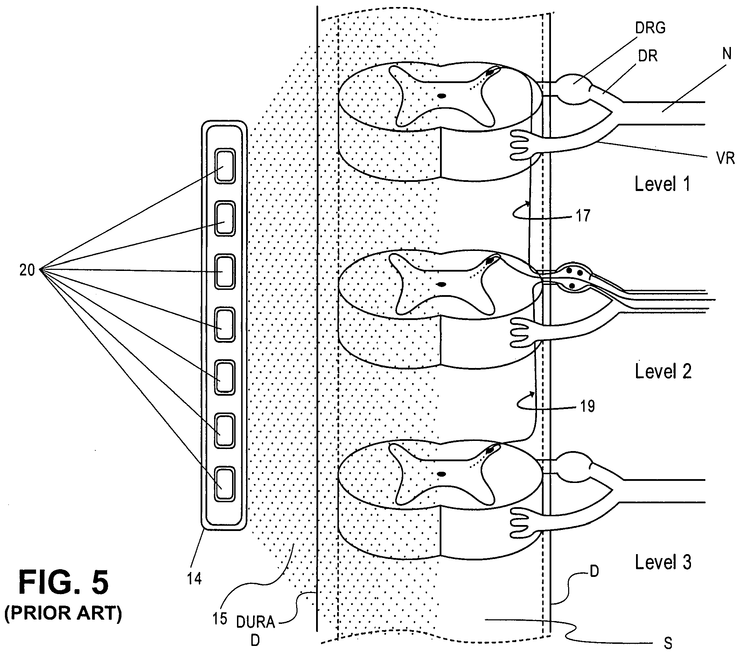

Although such SCS systems have effectively relieved pain in some patients, these systems have a number of drawbacks. To begin, as illustrated in Fig. 5, the lead 14 is positioned upon the spinal cord dura layer D so that the electrodes 20 stimulate a wide portion of the spinal cord and associated spinal nervous tissue. The spinal cord is a continuous body and three spinal levels of the spinal cord are illustrated. For purposes of illustration, spinal levels are sub-sections of the spinal cord S depicting that portion where the dorsal root DR and ventral root VR join the spinal cord S. The peripheral nerve N divides into the dorsal root DR and the dorsal root ganglion DRG and the ventral nerve root VR each of which feed into the spinal cord S. An ascending pathway 17 is illustrated between level 2 and level 1 and a descending pathway 19 is illustrated from level 2 to level 3. Spinal levels can correspond to the veterbral levels of the spine commonly used to describe the vertebral bodies of the spine. For simplicity, each level illustrates the nerves of only one side and a normal anatomical configuration would have similar nerves illustrated in the side of the spinal cord directly adjacent the lead.

Motor spinal nervous tissue, or nervous tissue from ventral nerve roots, transmits muscle/motor control signals. Sensory spinal nervous tissue, or nervous tissue from dorsal nerve roots, transmit pain signals. Corresponding dorsal and ventral nerve roots depart the spinal cord "separately"; however, immediately thereafter, the nervous tissue of the dorsal and ventral nerve roots are mixed, or intertwined. Accordingly, electrical stimulation by the lead 14 often causes undesirable stimulation of the motor nerves in addition to the sensory spinal nervous tissue.

Because the electrodes span several levels the generated stimulation energy 15 stimulates or is applied to more than one type of nerve tissue on more than one level. Moreover, these and other conventional, non-specific stimulation systems also apply stimulation energy to the spinal cord and to other neural tissue beyond the intended stimulation targets. As used herein, non-specific stimulation refers to the fact that the stimulation energy is provided to all spinal levels including the nerves and the spinal cord generally and indiscriminately. Even if the epidural electrode is reduced in size to simply stimulate only one level, that electrode will apply stimulation energy indiscriminately to everything (i.e. all nerve fibers and other tissues) within the range of the applied energy. Moreover, larger epidural electrode arrays may alter cerebral spinal fluid flow thus further altering local neural excitability states.

Another challenge confronting conventional neurostimulation systems is that since epidural electrodes must apply energy across a wide variety of tissues and fluids (i.e. CSF fluid amount varies along the spine as does pia mater thickness) the amount of stimulation energy needed to provide the desired amount of neurostimulation is difficult to precisely control. As such, increasing amounts of energy may be required to ensure sufficient stimulation energy reaches the desired stimulation area. However, as applied stimulation energy increases so too increases the likelihood of deleterious damage or stimulation of surrounding tissue, structures or neural pathways.
US 2006/0052839 discloses a system for stimulating a dorsal route ganglion comprising electrode leads and a controller. US-A-2006/0206118 describes percutaneous endoscopic access tools for the spinal epidural space and related methods of treatment.

Improved stimulation systems and methods are desired that enable more precise and effective delivery of stimulation energy.

### BRIEF SUMMARY OF THE INVENTION

Aspects of the present invention are set out in the appended independent claims, and preferred embodiments are set out in the appended dependent claims. As described above, the spinal cord is a continuous body and may be considered to include various spinal levels. For example, a spinal level may be considered a sub-section of the spinal cord wherein a dorsal root and ventral root join the spinal cord. Spinal levels may also correspond to vertebral levels of the spine commonly used to describe the vertebral bodies of the spine.

The target locations are stimulated individually, in contrast to conventional SCS leads which blanketly stimulate a wide area. This provides more effective treatment of pain symptoms and reduces deleterious side effects. The present invention provides systems for such targeted stimulation at various spinal levels. In addition, some embodiments provide additional specificity within each targeted level.

The systems can stimulate the various spinal levels at the dorsal root ganglion DRG. Examples described herein will illustrate specific stimulation of the dorsal root ganglia of various levels, however the embodiments are not so limited.

Also described is a method for stimulating a plurality of dorsal root ganglia. In some treatments the method comprises positioning a lead within an epidural space, wherein the lead has a longitudinal axis and at least two electrodes disposed along the longitudinal axis, aligning the lead so that each of the at least two electrodes is disposed within a distance of one of the plurality of dorsal root ganglia which allows selective stimulation thereto; and electrically energizing the lead so as to provide selective stimulation to at least one of the plurality of dorsal root ganglia. In some instances, electrically energizing comprises electrically energizing the lead so as to provide selective stimulation to at least two dorsal root ganglia which are not adjacent to each other. In other instances, the lead provides selective stimulation to at least two dorsal root ganglia which are adjacent to each other. Various combinations of dorsal root ganglions may be stimulated simultaneously or in any pattern.

In some embodiments, the at least two electrodes comprise a series of electrodes disposed along the longitudinal axis. In such embodiments, electrically energizing the lead may comprise selectively energizing individual electrodes within the series of electrodes which are disposed within the distance which allows selective stimulation to the associated dorsal root ganglion. It may be appreciated that at least some distances between the individual electrodes may be irregular.

In another example, a telescoping lead is provided for stimulation of a nerve within tissue of a body. In some embodiments, the telescoping lead comprises an elongate tubular body having a proximal end, a distal end, a lumen therethrough, and at least one electrode disposed thereon. The lead also includes an inner structure having a proximal end, a distal end, and at least one electrode disposed thereon, wherein the inner structure is advanceable through the lumen so that its distal end extends beyond the distal end of the elongate tubular body, and wherein the inner structure has a strength member extending between its proximal and distal ends so as to provide sufficient strength to allow tunneling of the lead through the tissue.

The tubular body and inner structure may have a variety of cross-sectional shapes. In some embodiments, the inner structure is shaped to resist rotation within the lumen. Or, the tubular body may be shaped to resist rotation around the inner structure. In some instances, the tubular body has an oval or oblong shaped lumen.

Typically, the electrodes are substantially longitudinally aligned. However, some electrodes may be adjacent to each other or longitudually offset from each other. Optionally, the electrodes may be individually energizable.

In some embodiments, the inner structure is sized for advancement through a foramen. Typically the tubular body would likewise be sized for such advancement.

In some embodiments, the telescoping lead further comprises an additional elongate tubular body having a proximal end, a distal end, a lumen therethrough, and at least one electrode disposed thereon. The additional elongate tubular body is configured to be advanceable through the lumen of the elongate tubular body and the inner structure is advanceable through the lumen of the additional elongate tubular body. Typically, the at least one electrode is substantially longitudinally aligned.

The elongate tubular body and inner structure may be positionable so that the at least one electrode on the elongate tubular body aligns with a first dorsal root ganglion while the at least one electrode on the inner structure aligns with a second dorsal root ganglion. Or, the elongate tubular body and inner structure are positionable so that the at least one electrode on the elongate tubular body aligns with a first portion of a dorsal root ganglion while the at least one electrode on the inner structure aligns with a second portion of the dorsal root ganglion.

Also described is a method for stimulating at lead toward a dorsal root ganglion, wherein the telescoping lead comprises an elongate tubular body having a proximal end, distal end, a lumen therethrough and at least one electrode disposed thereon, and an inner structure having at least one electrode disposed thereon, wherein the inner structure is advanceable through the lumen so that its distal end extends beyond the distal end of the elongate tubular body. The method further comprises positioning at least one of the at least one electrodes near the dorsal root ganglion so as to apply stimulation to the dorsal root ganglion.

In some embodiments, advancing the telescoping lead comprises advancing the telescoping lead at least partially through a foramen. Advancing the telescoping lead may optionally comprise laterally approaching the dorsal root ganglion from outside of a spinal column. Or, advancing the telescoping lead may comprise advancing the telescoping lead through an epidural space.

Typically, positioning comprises advancing or retracting the inner structure to position at least one of the at least one electrodes disposed on the inner structure near the dorsal root ganglion.

In some embodiments, positioning comprises advancing the inner structure so as to position at least one of the at least one electrodes disposed on the inner structure near the dorsal root ganglion and at least one of the at least one electrodes disposed on the tubular body near another dorsal root ganglion. The dorsal root ganglion and other dorsal root ganglion may be on adjacent spinal levels. However, it may be appreciated that the dorsal root ganglion and other dorsal root ganglion may not be on adjacent spinal levels.

Other objects and advantages of the present invention will become apparent from the detailed description to follow, together with the accompanying drawings.

### BRIEF DESCRIPTION OF THE DRAWINGS

Fig. 1A-1B, 2, 3, 4, 5 illustrate prior art.
Fig.6 illustrates positioning of a device of the present invention so as to optionally simultaneously stimulate various levels of the spinal cord.
Fig. 7 illustrates a device extending longitudinally through the foramens of each vertebrae.
Fig. 8 is a cross-sectional view of the device of Fig. 7.
Figs. 9A-9C illustrate embodiments of grouped lead devices that have electrodes at various spacings and/or allow adjustment of the spacing between the electrodes.
Fig. 10A illustrates an embodiment of a grouped lead device comprising a telescoping shaft.
Fig. 10B is a cross-sectional view of the device of Fig. 10A.
Fig. 10C is an alternative embodiment of the device of Fig. 10A having a generally circular cross-section.
Fig. 11 illustrates the telescoping shaft positioned so that the electrodes are near the DRGs.
Fig. 12 illustrates the stimulation of an individual DRG with a device having a plurality of selectively utilizable electrodes.
Fig. 13 illustrates the stimulation of an individual DRG with a device having a telescoping shaft.

### DETAILED DESCRIPTION OF THE INVENTION

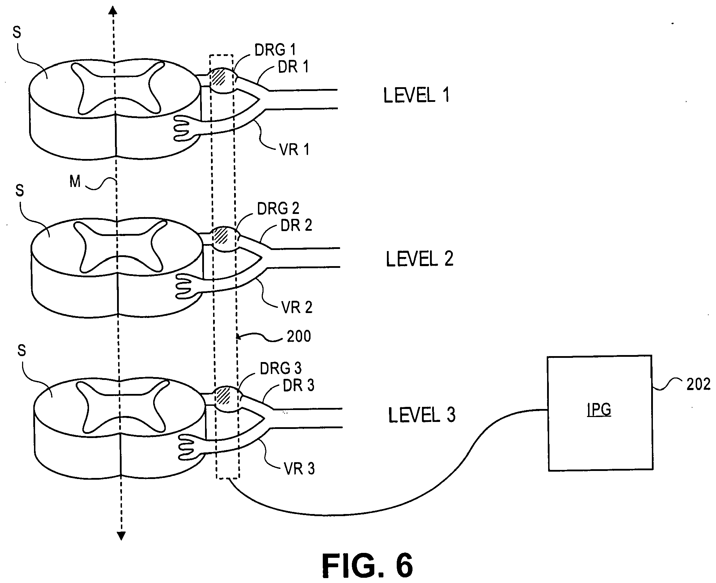

Fig. 6 illustrates positioning of an embodiment of a device 200 of the present invention so as to optionally simultaneously stimulate various levels (levels 1, 2, 3 in this example) of the spinal cord S. The device 200 is shown positioned within an epidural space of the spinal column at a lateral distance from the midline M of the of the spinal column which aligns the device 200 with the dorsal root ganglions DRG 1, DRG 2, DRG 3. Thus, portions of the device 220 align with and may optionally contact the dorsal root ganglions DRG 1, DRG 2, DRG 3, as indicated by shading. These aligned portions provide targeted or selective stimulation of one or more of the dorsal root ganglions DRG 1, DRG 2, DRG 3 while avoiding or reducing stimulation to surrounding tissues, such as the ventral roots VR1, VR2, VR3.

The device 200 is electrically connected to a power source or implantable pulse generator (IPG) 202, as shown, which is implanted in the body of the patient. Fig. 6 illustrates antegrade positioning of the device 200, however a retrograde approach may also be used.. Thus, the device 200 extends across multiple levels in the form of a grouped lead providing a single extension to the IPG 202, or any number of extensions which is less than the number of targeted levels.

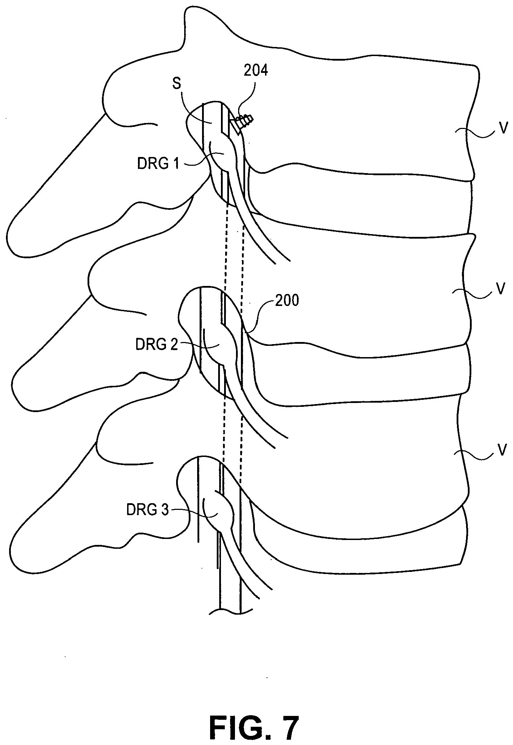

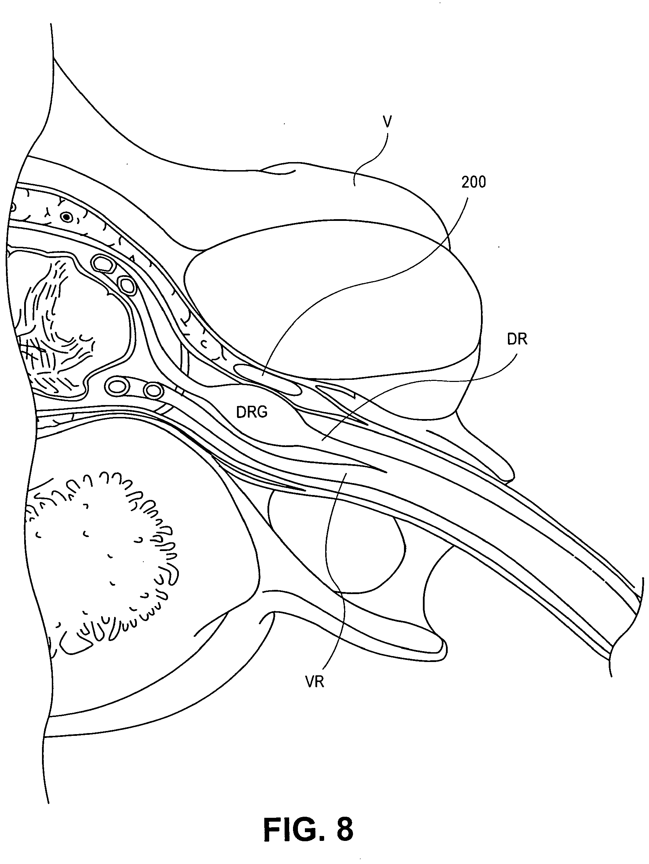

Fig. 7 provides a side view of the spinal cord S including the bony structures or vertebrae V which surround and protect the spinal cord S. The device 200 is shown extending longitudinally through the foramens of each vertebrae V. Optionally, the device 200 may be anchored to a vertebrae V with the use of an anchoring device 204, such as a bone screw or bone tack as shown, to resist migration. Fig. 8 provides a cross-sectional view of the device 200 of Fig. 7 showing the device 200 positioned against a DRG. It may be appreciated that the device 200 may be positioned at a variety of locations adjacent or near the DRG while maintaining the longitudinal orientation.

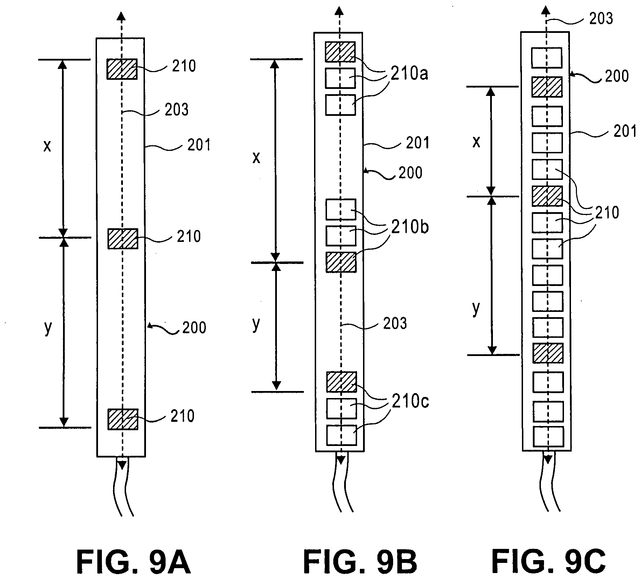

Figs. 9A-9C illustrate embodiments of devices 200 of the present invention that have electrodes 210 at various spacings and/or allow adjustment of the spacing between activated electrodes. Fig. 9A illustrates an embodiment of a device 200 comprising an elongate body 201 having at least two electrodes 210 disposed thereon. The elongate body 201 has a longitudinal axis 203, and in this embodiment, the at least two electrodes 210 are disposed along the longitudinal axis 203. The at least two electrodes 210 have a fixed spacing along the longitudinal axis 203 according to the distance between spinal levels or the longitudinal distance between DRGs along the spinal column (based on the average patient population or based on specific anatomies). Distances X & Y illustrate the distances between the at least two electrodes 210. Distances X & Y may be the same or different from each other. The distances are such that each electrode 210 is within range of a target DRG, when the device 200 is aligned according to Fig. 6, and each electrode 210 is disposed within a distance which allows selective stimulation thereto.

Fig. 9B illustrates an embodiment of a device 200 comprising an elongate body 201 having a plurality of electrodes 210 disposed thereon. The electrodes 210 are positioned so that two or more electrodes 210 grouped at spaced distances. For example, Fig. 9B illustrates a first group of electrodes 210a disposed near a distal end of the elongate body 201, a second group of electrodes 210b disposed midway along the elongate body 201, and a third group of electrodes 210c disposed near a proximal end of the elongate body 201. In this example, each group 210a, 210b, 210c includes three electrodes disposed along the longitudinal axis 203. When the elongate body 201 is positioned within the anatomy, slight anatomical variations in distance between DRGs may occur. Therefore, the electrode 210 within each group 210a, 210b, 210c that aligns most closely with the target DRG of that group or that provides the most desirable therapeutic effects may be utilized (as indicated by shading). The remaining two electrodes in the group will not receive stimulating energy. It may be appreciated that in some embodiments, more than one electrode 210 may be used within each group if such use provides a more desirable result. The distances X & Y between the stimulating electrodes can thus be varied to accommodate differences in DRG spacing for individual patients. Further, if by chance the device 200 migrates over time or is otherwise performing less than optimally, the electrodes 210 which receive stimulation energy can be changed without altering the position of the elongate body 201. Thus, the DRGs can be retargeted by modifying the distances X & Y.

Fig. 9C also illustrates an embodiment of a device 200 comprising an elongate body 201 having a plurality of electrodes 210 disposed thereon along a longitudinal axis 203. In this embodiment, the electrodes 210 are positioned substantially continuously along the device 200. Electrodes are selectively utilized (as indicated by shading) to adjust X & Y to accommodate differences in DRG spacing for individual patients. Any number of electrodes may be present in any arrangement. Also, any number of spaced distances may be present, such as to create distances such as X & Y & Z, etc.

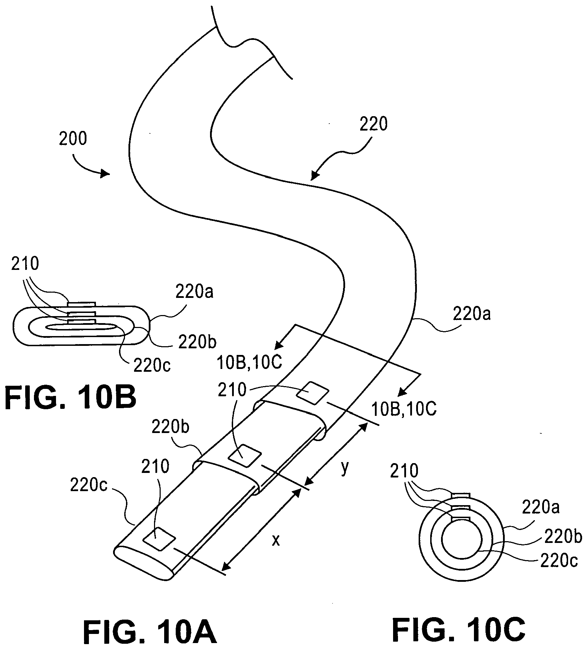

Figs. 10A-10C illustrate an embodiment of a grouped lead device 200 comprising a telescoping shaft 220 which allows adjustable positioning of the electrodes 210. In this embodiment, the telescoping shaft 220 comprises a first structure or elongate tubular body 220a and a second structure or elongate tubular body 220b. Each tubular body 220a, 220b has a proximal end, a distal end and a lumen therethrough. Each tubular body 220a, 220b also includes at least one electrode disposed thereon, typically near its distal end. In this embodiment, the second elongate tubular body is advanceable through the lumen of the first elongate tubular body 220a so that its distal end, and electrode 210 disposed thereon, extends beyond the distal end of the first elongate tubular body 220a. The shaft 220 also includes an inner structure 220c having a distal end and, wherein the inner structure is advanceable through the lumen of the second tubular body 220b so that its distal end extends beyond the distal end of the second elongate tubular body 220b. The inner structure 220c also has at least one electrode 210 disposed thereon, so that the electrode 210 is exposed beyond the distal end of the second elongate tubular body 220b. Thus, all three electrodes 210 may be simultaneously exposed and utilized for stimulating tissue. The distances X & Y between the electrodes 210 can be adjusted by moving the tubular bodies 220a, 220b and inner structure 220c relative to each other, such as by extension and retraction.

The telescoping structures 220a, 220b, 220c may be comprised of various materials, preferably a flexible polymer. In some embodiments, the inner structure 220c has a strength member extending between its proximal and distal ends so as to provide sufficient strength to allow tunneling of the device 200. Optionally, the structures 220a, 220b, 220c may be supported by a stylet during placement. The telescoping structures 220a, 220b, 220c may have various cross-sectional shapes, including shapes that resist rotation. Fig. 10B illustrates an example of a rotation resisting shape, a flat shape which may be oval or oblong, rectangular, polygonal, etc. The flatness of the shape resists rotation of, for example, the inner structure 220c within the second tubular body 220b or the second tubular body 220b within the first tubular body 220a. Such resistance rotation ensures that the electrodes maintain rotational orientation to each other, such as longitudinal alignment. Alternatively, the cross-sectional shape may be thick (Fig. 10C) which may be circular, square, rectangular, polygonal, etc. When the shape is circular, the rotational orientation of the electrodes can be adjusted.

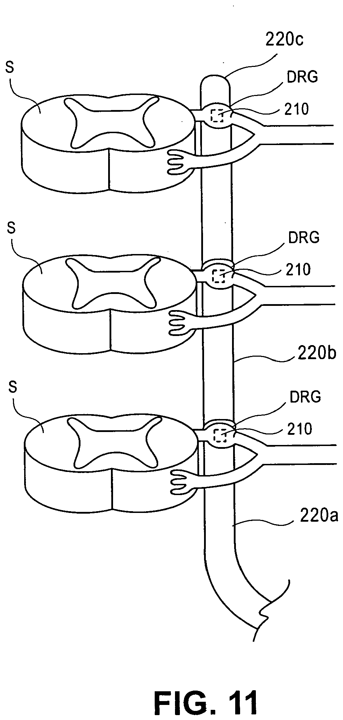

Electrodes 210 may be easier to attach to flat designs, conserve energy, etc. Flat designs may also provide easier determination of orientation of the electrodes 210, as describe above, during delivery and implantation. The cross-sectional shape may also be chosen based on location in anatomy where the device is to be placed. Fig. 11 illustrates the telescoping shaft 220 positioned within the epidural space so that the electrodes 210 are near DRGs on multiple spinal levels. This may be achieved by advancing or retracting the telescoping structures 220a, 220b, 220c so that the electrodes 210 substantially align with the DRGs. Conductive wires electrically connected to the electrodes 210 extend out the proximal end to an IPG.

The above embodiments describe systems that directly stimulate the dorsal root, particularly the dorsal root ganglion (DRG), while minimizing or excluding undesired stimulation of other anatomies. In some embodiments, this allows access to multiple levels of the spinal column with the use of a single device. This reduces procedure complexity, time and recovery since a single access path is created rather than individual access paths to each level of the spinal column. These embodiments also have a reduced number of paths to an IPG. It may be appreciated that the systems of the present invention may also be used to stimulate other portions of the spinal anatomy or other anatomies.

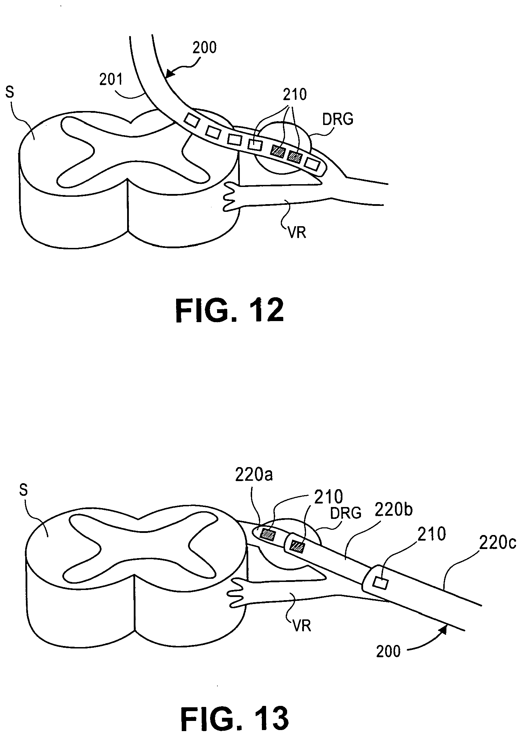

It may also be appreciated that the systems of the present invention may also be used to stimulate a single DRG. For example, Fig. 12 illustrates a device 200 such as or similar to the device of Fig. 9C wherein the device 200 comprises an elongate body 201 having a plurality of electrodes 210 disposed substantially continuously thereon along a longitudinal axis 203. The device 200 is advanced through the epidural space and at least a portion of the device 200 is advanced laterally toward a single DRG. In some instances, this includes advancement through a foramen. The device 200 is positioned so that at least one of the plurality of electrodes 210 is disposed on, near or about the DRG. The electrodes are then selectively utilized (as indicated by shading) so that the electrode(s) 210 which provide the most desirable result receive stimulation energy. The remaining electrodes receive a lower level or no stimulation energy.

Similarly, Fig. 13 illustrates a device 200 having a telescoping shaft 220 such as or similar to that of Fig. 10A. Again, the telescoping shaft 220 comprises a first structure or elongate tubular body 220a and a second structure or elongate tubular body 220b. Each tubular body 220a, 220b has a proximal end, a distal end and a lumen therethrough. Each tubular body 220a, 220b also includes at least one electrode disposed thereon, typically near its distal end. In this embodiment, the second elongate tubular body is advanceable through the lumen of the first elongate tubular body 220a so that its distal end, and electrode 210 disposed thereon, extends beyond the distal end of the first elongate tubular body 220a. The shaft 220 also includes an inner structure 220c having a distal end and, wherein the inner structure is advanceable through the lumen of the second tubular body 220b so that its distal end extends beyond the distal end of the second elongate tubular body 220b. The inner structure 220c also has at least one electrode 210 disposed thereon, so that the electrode 210 is exposed beyond the distal end of the second elongate tubular body 220b. Thus, all three electrodes 210 may be simultaneously exposed and utilized for stimulating tissue.

Fig. 13 shows the device 200 laterally approaching an individual DRG from outside of a spinal column. One or more of the first tubular body 220a, second tubular body 220b and inner structure 220c may be advanced or retracted so that at least one electrode 210 is disposed on, near or about the DRG. The electrodes are then selectively utilized (as indicated by shading) so that the electrode(s) 210 which provide the most desirable result receive stimulation energy. The remaining electrodes receive a lower level or no stimulation energy.

Although the foregoing invention has been described in some detail by way of illustration and example, for purposes of clarity of understanding, it will be obvious that various alternatives and modifications may be used and the above description should not be taken as limiting in scope of the invention.

## Claims

1. A system for selectively stimulating a Dorsal Root Ganglion and other portions of the spinal anatomy, the system comprising:
a telescoping lead (200) comprising an elongate body with a longitudinal axis (203), the body having at least two electrodes (210) disposed along the longitudinal axis configured such that the longitudinal distance between a first electrode and a second electrode is adjustable such that the first electrode is disposed on, near or about a Dorsal Root Ganglion to provide selective stimulation to the Dorsal Root Ganglion, and the second electrode is positioned outside of the Dorsal Root Ganglion to provide selective stimulation to a part of the spinal anatomy outside of the Dorsal Root Ganglion; and
an implantable pulse generator (202) connectable with the telescoping lead and configured to selectively provide stimulation energy to at least said first and second electrodes.

2. A system as claimed in claim 1, wherein the electrodes are individually energizeable.

3. A system as claimed in either of claims 1 or 2, wherein the electrode(s) which do not provide the most desirable result for selectively stimulating the Dorsal Root Ganglion, receive lower stimulation energy.

4. A system as claimed in claim 3, wherein the second electrode receives lower stimulation energy than the first electrode.

5. A system as claimed in any preceding claim, wherein the electrode(s) which do not provide the most desirable result for selectively stimulating the Dorsal Root Ganglion, receive no stimulation energy.

6. A system as claimed in any preceding claim, wherein the at least two electrodes are part of a group of electrodes disposed along the longitudinal axis of the elongate body, the elongate body comprises two or more groups of electrodes and each group of electrodes is spaced apart from the next according to the distance between Dorsal Root Ganglions along the spinal column.

7. A system as claimed in any preceding claim, wherein the telescoping lead is sized for advancement through an epidural space of the body.

8. A system as claimed in any preceding claim, wherein the telescoping lead is sized for advancement laterally toward the dorsal root ganglion.

9. A system as claimed in any preceding claim, wherein the telescoping lead is configured for advancement through a foramen.

## Patentansprüche

1. System zur selektiven Stimulation eines Spinalganglions und von anderen Abschnitten der spinalen Anatomie, wobei das System Folgendes umfasst:
eine teleskopische Leitung (200), umfassend einen länglichen Körper mit einer Längsachse (203), wobei der Körper mindestens zwei Elektroden (210) aufweist, die entlang der Längsachse angeordnet sind, konfiguriert dergestalt, dass der Längsabstand zwischen einer ersten Elektrode und einer zweiten Elektrode dergestalt justierbar ist, dass die erste Elektrode an einem, in der Nähe von einem oder um ein Spinalganglion herum zur Bereitstellung einer selektiven Stimulation an dem Spinalganglion angeordnet ist, und die zweite Elektrode auf der Außenseite des Spinalganglions zur Bereitstellung einer selektiven Stimulation an einem Teil der spinalen Anatomie auf der Außenseite des Spinalganglions positioniert ist; und
einen implantierbaren Impulsgenerator (202), der mit der teleskopischen Leitung verbindbar ist und zur selektiven Bereitstellung von Stimulationsenergie an mindestens die genannten ersten und zweiten Elektroden konfiguriert ist.

2. System nach Anspruch 1, wobei die Elektroden individuell erregbar sind.

3. System nach einem der beiden Ansprüche 1 oder 2, wobei die Elektrode(n), die nicht das wünschenswerteste Ergebnis zur selektiven Stimulation des Spinalganglions bereitstellt/bereitstellen, eine niedrigere Stimulationsenergie empfängt/empfangen.

4. System nach Anspruch 3, wobei die zweite Elektrode eine niedrigere Stimulationsenergie empfängt als die erste Elektrode.

5. System nach einem der vorangehenden Ansprüche, wobei die Elektrode(n), die nicht das wünschenswerteste Ergebnis zur selektiven Stimulation des Spinalganglions bereitstellt/bereitstellen, keine Stimulationsenergie empfängt/empfangen.

6. System nach einem der vorangehenden Ansprüche, wobei die mindestens zwei Elektroden Teil einer Gruppe von Elektroden sind, die entlang der Längsachse des länglichen Körpers angeordnet sind, der längliche Körper zwei oder mehr Gruppen von Elektroden umfasst und jede Gruppe von Elektroden von der nächsten je nach Abstand zwischen den Spinalganglien entlang der Wirbelsäule beabstandet ist.

7. System nach einem der vorangehenden Ansprüche, wobei die teleskopische Leitung zum Vorschub durch einen Epiduralraum des Körpers bemessen ist.

8. System nach einem der vorangehenden Ansprüche, wobei die teleskopische Leitung zum Vorschub seitlich auf das Spinalganglion ausgerichtet bemessen ist.

9. System nach einem der vorangehenden Ansprüche, wobei die teleskopische Leitung zum Vorschub durch ein Foramen konfiguriert ist.

## Revendications

1. Système de stimulation sélective d'un ganglion rachidien et d'autres parties de l'anatomie spinale, le système comprenant :
un câble télescopique (200) comprenant un corps allongé ayant un axe longitudinal (203), le corps comportant au moins deux électrodes (210) disposées le long de l'axe longitudinal, configurées de telle sorte que la distance longitudinale entre une première électrode et une deuxième électrode soit réglable de telle sorte que la première électrode soit disposée sur, à proximité ou autour d'un ganglion rachidien pour produire une stimulation sélective du ganglion rachidien, et que la deuxième électrode soit positionnée à l'extérieur du ganglion rachidien pour produire une stimulation sélective d'une partie de l'anatomie spinale à l'extérieur du ganglion rachidien ; et
un générateur d'impulsions implantable (202) qui peut être raccordé au câble télescopique et configuré pour apporter de manière sélective une énergie de stimulation au moins auxdites première électrode et deuxième électrode.

2. Système selon la revendication 1, dans lequel les électrodes peuvent être excitées individuellement.

3. Système selon l'une ou l'autre des revendications 1 ou 2, dans lequel l'électrode ou les électrodes qui ne produisent pas le résultat le plus souhaitable pour la stimulation sélective du ganglion rachidien reçoivent une énergie de stimulation plus basse.

4. Système selon la revendication 3, dans lequel la deuxième électrode reçoit une énergie de stimulation plus basse que la première électrode.

5. Système selon l'une quelconque des revendications précédentes, dans lequel l'électrode ou les électrodes qui ne produisent pas le résultat le plus souhaitable pour la stimulation sélective du ganglion rachidien ne reçoivent aucune énergie de stimulation.

6. Système selon l'une quelconque des revendications précédentes, dans lequel lesdites au moins deux électrodes font partie d'un groupe d'électrodes disposées le long de l'axe longitudinal du corps allongé, le corps allongé comprend deux ou plusieurs groupes d'électrodes, et chaque groupe d'électrodes est espacé du groupe suivant en fonction de la distance qui sépare les ganglions rachidiens le long de la colonne vertébrale.

7. Système selon l'une quelconque des revendications précédentes, dans lequel le câble télescopique est dimensionné pour avancer à travers un espace épidural du corps.

8. Système selon l'une quelconque des revendications précédentes, dans lequel le câble télescopique est dimensionné pour avancer latéralement vers le ganglion rachidien.

9. Système selon l'une quelconque des revendications précédentes, dans lequel le câble télescopique est configuré pour avancer à travers un foramen.
